# EUROPEAN PATENT APPLICATION

(11) **EP 2 980 210 A1**
(43) Date of publication of application: **03.02.2016**
(21) Application number: 15178464.2
(22) Date of filing: 27.07.2015
(51) Int. Cl.: C12N 9/24

(54) **THERMOSTABLE XYLANASE BELONGING TO GH FAMILY 10**

(30) Priority: 28.07.2014 JP 2014153337
(71) Applicant: HONDA MOTOR CO., LTD., Tokyo, 107-8556 (JP)
(72) Inventor: Suda, Migiwa, Saitama, 351-0188 (JP); Okuma, Jiro, Saitama, 351-0188 (JP); Hirose, Yoshitsugu, Saitama, 351-0188 (JP); Yamaguchi, Asuka, Saitama, 351-0188 (JP); Kondo, Yasuhiro, Saitama, 351-0188 (JP); Kato, Tomohiko, Chiba, 292-0818 (JP); Shibata, Daisuke, Chiba, 292-0818 (JP)
(74) Representative: Beder, Jens

(57) **Abstract**

A thermostable xylanase including a xylanase catalytic domain, the xylanase catalytic domain including:
(A) a polypeptide comprising an amino acid sequence represented by SEQ ID NO:1;
(B) a polypeptide including an amino acid sequence in which at least one amino acid is deleted, substituted, or added in the amino acid sequence represented by SEQ ID NO: 1, and having xylanase activity at least under conditions of a temperature of 90°C and a pH of 6.0; or
(C) a polypeptide including an amino acid sequence having at least 75% sequence identity with the amino acid sequence represented by SEQ ID NO: 1, and having xylanase activity at least under conditions of a temperature of 90°C and a pH of 6.0.

### [Sequence Listing]

SEQUENCE LISTING

<110>  Honda Motor Co., Ltd.

<120>  Thermostable GH10 xylanase

<130>  P47809/EP

<140>
<141>

<150>  JP 2014-153337
<151>  2014-07-28

<160>  6
<170>  PatentIn version 3.1

<210>  1
<211>  357
<212>  PRT
<213>  Unknown

<220>
<223>  AR19G-10-6

<400>  1
Met Lys Leu Tyr Arg Tyr Gly Leu Pro Leu Leu Val Val Cys Val Ser
1               5                   10                  15
Trp Phe Ala Ser Leu Val Leu Gly Gln Val Asp Ser Val Thr Gln Leu
            20                  25                  30
Gly Pro Leu Arg Ser Leu Ala Val Arg Ala Gly Ile Glu Phe Gly Thr
        35                  40                  45
Ala Val Ser Ala Ser His Leu Asp Glu Asp Ser Val Tyr Arg Thr Leu
    50                  55                  60
Leu Ser Arg Glu Phe Ser Ser Ile Thr Pro Glu Asn Glu Met Lys Trp
65                  70                  75                  80
Glu Ser Leu Glu Pro Glu Pro Gly Arg Tyr Val Trp Glu Asn Ala Asp
                85                  90                  95
Arg Ile Val Ala Phe Ala Arg Ala His Gln Gln Arg Ile Arg Gly His
            100                 105                 110
Thr Leu Val Trp His Asn Gln Met Pro Val Trp Leu Lys Pro Asp Ala
        115                 120                 125
Phe Ser Pro Asp Glu Leu Arg Lys Ile Leu Gln Arg His Ile Gln Thr
    130                 135                 140
Val Val Gly Arg Tyr Arg Gly Ser Ile Trp Gln Trp Asp Val Val Asn
145                 150                 155                 160
Glu Ala Phe Asn Glu Asp Gly Thr Phe Arg Asp Thr Leu Trp Tyr Arg
                165                 170                 175
Ala Phe Gly Gly Pro Glu Tyr Ile Ala Trp Ala Phe Arg Trp Ala Lys
            180                 185                 190
Glu Ala Asp Pro Gln Ala Leu Leu Phe Tyr Asn Asp Tyr Asn Leu Glu
        195                 200                 205
Phe Pro Ser Pro Lys Ala Asp Ala Val Leu Ser Phe Leu Ala Arg Leu
    210                 215                 220
Lys Ala Glu Gly Val Pro Ile Asp Gly Ile Gly Leu Gln Gly His Ile
225                 230                 235                 240
Ser Leu Arg Tyr Gly Phe Pro His Ala Gln Glu Leu Leu Asp His Val
                245                 250                 255
Arg Arg Phe Val Asp Ala Gly Phe Tyr Val Ala Phe Thr Glu Val Asp
            260                 265                 270
Val Arg Met Pro Leu Pro Arg Asp Asn Thr Lys Asp Leu Ala Gln Ala
        275                 280                 285
Gln Val Tyr Thr Gln Ile Leu Ser Thr Cys Leu Ala Leu Lys Gly Lys

290                 295                 300
Cys Leu Ser Phe Ser Ile Trp Gly Phe Pro Asp Lys Tyr Ser Trp Val
305                 310                 315                 320
Pro Gly Phe Phe Pro Gly Glu Gly Ala Ala Thr Pro Ile Ala Asp Asp
                325                 330                 335
Tyr Ser Pro Lys Pro Ala Trp Tyr Gln Met Gly Leu Leu Leu Tyr Val
            340                 345                 350
Ala Ala Lys Gly Arg
        355

<210>  2
<211>  1074
<212>  DNA
<213>  Unknown

<220>
<223>  Open reading frame AR19G-10

<400>  2
atgaagctct atcggtacgg cttgccccta ttagttgtgt gcgtttcctg gttcgcttcc      60
ctagtcttag ggcaggtgga ctcggtaacc cagttaggtc cattgaggtc gcttgctgtg     120
cgagcaggca tagagtttgg cacagctgtt tctgcatctc atttggatga agattctgtt     180
tatcgcacgc ttctgagtcg cgagttttcg tctattaccc ctgaaaacga aatgaagtgg     240
gagagcttgg agcctgagcc agggcggtat gtctgggaaa acgctgatcg catagttgct     300
tttgctcgtg ctcatcagca aaggatacga ggccacacgt tggtatggca taatcagatg     360
cctgtttggc tcaagccaga tgctttttcg cccgacgagc tccgtaaaat cctccaaagg     420
catattcaaa cggtggttgg gaggtatagg ggaagtattt ggcagtggga tgtagtcaac     480
gaggctttca atgaggatgg tacgtttcgc gataccettt ggtatagagc ttttgggggc     540
ccagagtata ttgcttgggc tttccgttgg gcgaaggagg cagaccccca ggctctgcta     600
ttttataatg actacaatct agaattccct tcgcctaagg ctgatgctgt cctctccttc     660
ttagcacgcc ttaaagcaga gggggtgccc attgacggta taggcctcca aggacatatc     720
agtctgcgtt atggttttcc gcatgcccaa gagttgctgg accacgtgcg gcggtttgtg     780
gatgcgggtt tttacgtagc ttttacggag gttgatgtgc gcatgcccct tcctcgcgat     840
aacacaaaag acttggcaca agcccaggtg tatacccaga ttttgagtac gtgcctggcc     900
ctaaagggga agtgcctctc cttcagcata tggggttttc cagacaaata ctcttgggtt     960
ccaggatttt tccctgggga aggagccgca actccaatcg cggatgatta tagcccgaag    1020
ccggcttggt atcagatggg gcttcttctt tatgtagccg ccaaagggag gtag          1074

<210>  3
<211>  24
<212>  DNA
<213>  Artificial Sequence

<220>
<223>  Description of Artificial Sequence: Primer.

<400>  3
atgaagctct atcggtacgg cttg                                             24

<210>  4
<211>  24
<212>  DNA
<213>  Artificial Sequence

<220>
<223>  Description of Artificial Sequence: Primer.

<400>  4
ctacctccct ttggcggcta cata                                             24

<210>  5
<211>  28
<212>  DNA

<213>  Artificial Sequence

<220>
<223>  Description of Artificial Sequence: Primer.

<400>  5
caccatgaag ctctatcggt acggcttg                                          28

<210>  6
<211>  361
<212>  PRT
<213>  Streptomyces hygroscopicus subsp. jinggangensis 5008

<220>
<223>  GH10 xylanase

<400>  6
Met Asn Leu Pro Arg Ser Leu Ala Ala Val Ala Ser Val Cys Cys Leu
1               5                   10                  15
Thr Leu Leu Pro Leu Val Ala Ala Ser Pro Ala Ser Ala Phe Gly Gly
            20                  25                  30
Thr Gly Val Pro Asp Arg Pro Leu Arg Gln Leu Ala Ala Asp Asp His
        35                  40                  45
Leu Arg Val Gly Thr Ala Val Asp Met Ser Ala Leu Ala Asp Asp Ala
    50                  55                  60
Ala Tyr Arg Arg Leu Ala Gly Ser Glu Phe Ser Thr Val Thr Pro Glu
65                  70                  75                  80
Asn Val Met Lys Trp Glu Ala Ile Glu Pro Arg Gln Gly Glu Tyr Asp
                85                  90                  95
Tyr Ala Pro Ala Asp Arg Leu Val Asp Phe Ala Arg Lys His Gly Gln
            100                 105                 110
Lys Val Arg Gly His Val Leu Val Trp His Ser Gln Leu Pro Ser Trp
        115                 120                 125
Leu Thr Ser Gly Asp Phe Ser Ala Asp Gln Leu Arg Glu Ile Leu His
    130                 135                 140
Arg His Ile Thr Asp Thr Val Arg His Phe Lys Gly Arg Val Trp Gln
145                 150                 155                 160
Trp Asp Val Val Asn Glu Ala Phe Asn Glu Asp Gly Thr Leu Arg Asp
                165                 170                 175
Ser Ile Trp Leu Arg Glu Leu Gly Pro Gly Tyr Ile Ala Asp Ala Phe
            180                 185                 190
Arg Trp Ala His Glu Ala Asp Pro His Ala Leu Leu Phe Tyr Asn Asp
        195                 200                 205
Tyr Asn Thr Glu Trp Thr Gly Pro Lys Ser Asp Ala Val Tyr Glu Leu
    210                 215                 220
Ala Gly Arg Leu Arg Ala Gln Gly Val Pro Ile Asp Gly Val Gly Phe
225                 230                 235                 240
Gln Gly His Leu Gly Ile Gln Tyr Gly Leu Pro Ser Gly Met Ala Ala
                245                 250                 255
Asn Phe Ala Arg Phe Asp Lys Leu Gly Leu Ala Thr Ala Val Thr Glu
            260                 265                 270
Ala Asp Val Arg Met Pro Leu Pro Ala Asp Ala Asp Lys Leu Ala Thr
        275                 280                 285
Gln Ala Glu Gly Tyr Arg Leu Leu Leu Asp Ala Cys Leu Arg Thr Arg
    290                 295                 300
His Cys Ile Ser Phe Thr Val Trp Gly Phe Thr Asp Thr Tyr Ser Trp
305                 310                 315                 320
Val Pro Gly Thr Phe Pro Gly Glu Gly Ala Ala Asn Leu Tyr Asp Glu
                325                 330                 335
Asp Tyr Arg Pro Lys Pro Ala Tyr Arg Thr Leu Gln Ser Ala Leu Ala
            340                 345                 350
Gly Arg Ala Arg Ala Gly Gly Ala Glu
        355                 360

## Description

### TECHNICAL FIELD

The present invention relates to a thermostable xylanase, a polynucleotide encoding the aforementioned thermostable xylanase, an expression vector for expressing the aforementioned thermostable xylanase, a transformant into which the aforementioned expression vector has been incorporated and a method for producing a lignocellulose degradation product using the aforementioned thermostable xylanase.

Priority is claimed on Japanese Patent Application No. 2014-153337, filed July 28, 2014, the content of which is incorporated herein by reference.

### BACKGROUND ART

In recent years, the development of alternative energy to oil is a very important issue, because of environmental problems, such as global warming and aerial pollution, in addition to the concern related to transportation energy supply. Plant biomass is the most abundant renewable energy source on earth, which is expected to serve as an alternative source to petroleum. The main components of plant biomass (lignocellulose) are polysaccharides such as celluloses and hemicelluloses (including xylan, arabinan and mannan), lignin and other pectins. These polysaccharides are hydrolyzed into monosaccharides such as glucose and xylose by a variety of glycoside hydrolases, and are used as a biofuel or a raw material of chemical products.

Lignocellulose is poorly degradable due to its highly complicated structures, and is hard to degrade or hydrolyze with a single cellulolytic enzyme. For this reason, the hydrolysis of cellulose among the polysaccharides generally requires three types of enzymes: an endoglucanase of glucoside hydrolase (endo-1,4-β-D-glucanase, EC 3.2.1.4), an exo-type cellobiohydrolase (1,4-β-cellobiosidase or cellobiohydrolase, EC 3.2.1.91, EC 3.2.1.176), and a β-glucosidase (EC 3.2.1.21). On the other hand, hemicellulose contains xylan, arabinan, mannan and the like, and the composition thereof depends on the type of the plants. For example, xylan is a major constituent in broad-leaved trees, herbaceous plants and the like. For the hydrolysis of xylan, it is thought that xylanase (endo-1,4-β-xylanase, EC 3.2.1.8) and β- xylosidase (EC 3.2.1.37) are required.

In the conventional lignocellulose to ethanol conversion process, high-solid loading up to 30-60% in initial substrate concentration has been attempted for the purpose of higher energy efficiency and less water usage. The enzymatic hydrolysis of lignocellulose by such high-solid loading results in the high viscosity of the hydrolyzed biomass solution so that the hydrolysis of lignocellulose hardly proceeds. Therefore, for example, by carrying out the enzymatic hydrolysis process at a high temperature of 80°C or higher using a thermostable enzyme, in addition to an increase in the hydrolysis reaction rate, since the viscosity of the hydrolyzed biomass solution also reduces, the shortening of the hydrolysis reaction time and the reduction of the amount of enzyme are expected to be achieved. For this reason, for various glycoside hydrolases, development of enzymes that are more excellent in terms of thermostability has been desired.

Many thermostable glycoside hydrolases have been obtained by isolating and identifying the thermophilic microorganisms that live in a high temperature environment, cloning the genes from these cultured and isolated microorganisms and determining the DNA sequence thereof, followed by the expression thereof using *E. coli,* filamentous fungi and the like. In particular, regarding xylanases required for the hydrolysis of xylan as a hemicellulose, for the purpose of lignocellulose degradation, pulp processing and the like, many of them have been isolated from the thermophilic bacteria, filamentous fungi, archaea and the like to date. For example, a xylanase derived from *Acidothermus cellulolyticus* that exhibits an enzyme activity at 60 to 80°C, a xylanase derived from anaerobic heat-resistant bacteria isolated from a hot spring in New Zealand that exhibits an enzyme activity at 60 to 80°C and a xylanase derived from bacteria belonging to the genus Bacillus isolated from the soil that has an optimum temperature of 80°C have been disclosed in Patent Document 1, Patent Document 2 and Patent Document 3, respectively. In Non-Patent Document 4, a xylanase derived from *Acidothermus cellulolyticus* that has an optimum temperature of around 60 to 80°C has been reported. In addition, attempts have also been made to further improve the heat resistance, and, for example, xylanases with improved heat resistance and the like which are obtained by replacing the amino acids of native enzyme have been described in Patent Documents 4 to 7. In Patent Document 8, it has been described that it is possible to increase the amount of enzyme production in the host by deleting a carbohydrate binding module, or the carbohydrate binding module and a linker from a native-type xylanase in which between the enzyme active domain and the carbohydrate binding module is connected by the linker. Most of these enzymes have an optimum temperature of 60 to 80°C, and further increase in the degree of heat resistance has been desired.

On the other hand, as extremely thermostable xylanases, in Patent Documents 9 and 10 and Non-Patent Documents 1 to 3, xylanases isolated from certain bacteria and filamentous fungi with an optimum temperature exceeding 85°C have been reported. According to Patent Document 9, a xylanase derived from *Rhodothermus marinus* with an optimum temperature of 90°C has been reported, although the specific activity is about 26 nkat / mg protein (= about 1.6U / mg protein) at 90°C, which is rather low.

### PRIOR ART DOCUMENTS

### [PATENT DOCUMENTS]

[Patent Document 1] United States Patent No. 5,902,581
[Patent Document 2] United States Patent No. 6,083,733
[Patent Document 3] Japanese Unexamined Patent Application, First Publication No. 2004-121257
[Patent Document 4] United States Patent No. 5,759,840
[Patent Document 5] United States Patent No. 5,866,408
[Patent Document 6] United States Patent No. 7,060,482
[Patent Document 7] United States Patent Application Publication No. 2010/0062511
[Patent Document 8] United States Patent Application Publication No. 2006/0014247
[Patent Document 9] United States Patent No. 5,395,765
[Patent Document 10] United States Patent No. 5,688,668

### [Non-Patent Documents]

[Non-Patent Document 1] ZHENG QIANG ET AL., JOURNAL OF BIOSCIENCE AND BIOENGINEERING, 2001, VOL. 92, P. 423-428.
[Non-Patent Document 2] WINTERHALTER AND LIEBL, APPLIED AND ENVIRONMENTAL MICROBIOLOGY, 1995, VOL. 61, P. 1810-1815.
[Non-Patent Document 3] YOON ET AL, AGRICULTURAL CHEMISTRY AND BIOTECHNOLOGY, 2004, VOL. 47, P. 157-160.
[Non-Patent Document 4] KISHISHITA ET AL., PROTEIN EXPRESSION AND PURIFICATION, 2014, VOL. 94, P. 40-45.

### [Summary of the Invention]

### [Problems to be Solved by the Invention]

The present invention has an object of providing a novel thermostable xylanase exhibiting xylanase activity at least under conditions of a temperature of 90°C and a pH of 6.0, a polynucleotide encoding the aforementioned thermostable xylanase, an expression vector for expressing the aforementioned thermostable xylanase, a transformant into which the aforementioned expression vector has been incorporated and a method for producing a lignocellulose degradation product using the aforementioned thermostable xylanase.

### [Means for Solving the Problem]

In order to solve the above-mentioned problems, the inventors of the present invention have successfully obtained thermostable xylanases having novel amino acid sequences by extracting DNA directly from hot spring high temperature soils and conducting large-scale metagenome sequencing of hardly culturable microbiota. This has led to the completion of the present invention.

That is, as the thermostable xylanase, polynucleotide, expression vector, transformant, method for producing a thermostable xylanase, glycoside hydrolase mixture and method for producing a lignocellulose degradation product according to the present invention, the following aspects [1] to [10] can be mentioned.
[1] A thermostable xylanase including a xylanase catalytic domain, the xylanase catalytic domain including:
   (A) a polypeptide including an amino acid sequence represented by SEQ ID NO: 1;
   (B) a polypeptide including an amino acid sequence in which at least one amino acid is deleted, substituted, or added in the amino acid sequence represented by SEQ ID NO: 1, and having xylanase activity at least under conditions of a temperature of 90°C and a pH of 6.0; or
   (C) a polypeptide including an amino acid sequence having at least 75% sequence identity with the amino acid sequence represented by SEQ ID NO: 1, and having xylanase activity at least under conditions of a temperature of 90°C and a pH of 6.0.
[2] The thermostable xylanase according to the aforementioned aspect [1], which exhibits xylanase activity in a pH of 6.0 and a temperature of 60 to 99°C.
[3] A polynucleotide including a region that encodes a xylanase catalytic domain which includes:
   (a) a nucleotide sequence that encodes a polypeptide including an amino acid sequence represented by SEQ ID NO: 1;
   (b) a nucleotide sequence that encodes a polypeptide including an amino acid sequence in which at least one amino acid is deleted, substituted, or added in the amino acid sequence represented by SEQ ID NO: 1, and having xylanase activity at least under conditions of a temperature of 90°C and a pH of 6.0;
   (c) a nucleotide sequence that encodes a polypeptide including an amino acid sequence having at least 75% sequence identity with the amino acid sequence represented by SEQ ID NO: 1, and having xylanase activity at least under conditions of a temperature of 90°C and a pH of 6.0;
   (d) a nucleotide sequence having at least 75% sequence identity with a nucleotide sequence represented by SEQ ID NO: 2, and encoding a polypeptide having xylanase activity at least under conditions of a temperature of 90°C and a pH of 6.0; or
   (e) a nucleotide sequence of a polynucleotide which hybridizes with a polynucleotide including a nucleotide sequence represented by SEQ ID NO: 2 under a stringent condition, and being a nucleotide sequence that encodes a polypeptide having xylanase activity at least under conditions of a temperature of 90°C and a pH of 6.0.
[4] The polynucleotide according to the aforementioned aspect [3], wherein the aforementioned polypeptide exhibits xylanase activity in a pH of 6.0 and a temperature of 60 to 99°C.
[5] An expression vector, which is incorporated with the polynucleotide according to the aforementioned aspect [3] or [4], and
   which is able to express a polypeptide having xylanase activity in a host cell.
[6] A transformant, which is introduced with the expression vector according to the aforementioned aspect [5].
[7] The transformant according to the aforementioned aspect [6], which is a eukaryotic microbe.
[8] A method for producing a thermostable xylanase, the method including a step of producing a thermostable xylanase in the transformant according to the aforementioned aspect [6] or [7].
[9] A glycoside hydrolase mixture, including the thermostable xylanase according to the aforementioned aspect [1] or [2], a thermostable xylanase encoded by the polynucleotide according to the aforementioned aspect [3] or [4], or a thermostable xylanase produced by the method for producing a thermostable xylanase according to the aforementioned aspect [8], and at least one or more types of other glycoside hydrolases.
[10] A method for producing a lignocellulose degradation product, the method including producing a lignocellulose degradation product by bringing a material composed of lignocellulose containing hemicellulose including xylan into contact with the thermostable xylanase according to the aforementioned aspect [1] or [2], a thermostable xylanase encoded by the polynucleotide according to the aforementioned aspect [3] or [4], the transformant according to the aforementioned aspect [6] or [7], a thermostable xylanase produced by the method for producing a thermostable xylanase according to the aforementioned aspect [8], or the glycoside hydrolase mixture according to the aforementioned aspect [9].

### [Effects of the Invention]

The thermostable xylanase according to the present invention has xylanase activity at least under conditions of a temperature of 90°C and a pH of 6.0. For this reason, the aforementioned thermostable xylanase is suitable for a hydrolysis process of hemicellulose including xylan in high temperature conditions.

In addition, the polynucleotide, the expression vector into which the aforementioned polynucleotide has been incorporated and the transformant into which the aforementioned expression vector has been introduced according to the present invention are suitably used for the production of the thermostable xylanase according to the present invention.

### BRIEF DESCRIPTION OF THE DRAWINGS

FIG. 1 is an alignment representation of the amino acid sequence (SEQ ID NO: 1) of a polypeptide (AR19G-10-6) encoded by a gene clone AR19G-10-6 and the amino acid sequence of a GH10 xylanase (SEQ ID NO: 6) of *Streptomyces hygroscopicus subsp. jinggangensis* 5008 belonging to the phylum Actinobacteria.
FIG. 2 is a diagram showing the results of SDS-PAGE analysis of the AR19G-10-6 protein obtained by expressing the AR19G-10-6 gene in *Escherichia coli* in Example 1.
FIG. 3 is a diagram showing the measurement results of the xylanase activity of the AR19G-10-6 protein expressed in E. coli in Example 1 for each substrate.
FIG. 4 is a diagram showing the results of the xylanase activity (pH 6.0) of the AR19G-10-6 protein expressed in *E. coli* measured at respective temperatures in Example 1.
FIG. 5 is a diagram showing the results of the xylanase activity (90°C) of the AR19G-10-6 protein expressed in *E. coli* measured at respective pH values in Example 1.
FIG. 6 is a diagram showing a change in the fluorescence intensity of SYPRO Orange caused in association with the thermal denaturation exhibited by the AR19G-10-6 protein expressed in *E. coli* in Example 1.

### DESCRIPTION OF THE EMBODIMENT

### [Thermostable xylanase]

Many microorganisms including filamentous fungi, bacteria and archaea are difficult to culture, and about 99% of the microorganisms living in the microbial environments such as soil is said to be unknown microbes. In particular, the culturing of microorganisms living in a high temperature environment is extremely difficult, and it is thought that merely 0.1% or less of the microorganisms living in soils have been isolated and cultured with the currently available microbial culturing techniques. This difficulty to culture such microorganisms living in high temperature soils is one factor to hinder the development of thermostable enzymes.

In recent years, because of the development of the next generation giga sequencer enabling large amount sequencing of giga base pairs, it has become possible to conduct the whole genome sequencing of the microbial flora contained in soil and the like. Using this analysis technology, the metagenomic analysis method has been proposed in which the genome DNA of a microbial group is prepared from an environmental sample such as soil, the genomes of the group having nonuniform and miscellaneous genomic organizations are directly and comprehensively sequenced, and the sequenced data are assembled by a parallel computer, so as to thereby reconstruct the genomic sequences of the microbiota. This has contributed to the rapid progress in the genome sequencing of hardly culturable microorganisms.

As shown in Example 1 described later, the inventors of the present invention extracted the genomic DNA (metagenomic DNA) of the microbial groups collected from high temperature hot spring soils (for example, hot spring water of 58 to 78°C that contains soil, mud, microbial mats, biofilms and the like may be mentioned), and conducted shotgun sequencing and annotation of the metagenomic DNA.

By so doing, open reading frames (ORFs) encoding the amino acid sequences similar to known xylanase enzymes (for example, amino acid sequences having 20% or higher sequence identity and Expectation value (i.e. E-value) is less than 1e⁻²⁰) were obtained.

Primers were designed based on the nucleotide sequence information of 33 ORFs in which the presence of xylanase catalytic domain could be verified, and gene candidates were cloned from the metagenomic DNA of the high temperature hot spring soils by the PCR method. The PCR-cloned DNAs were incorporated in *E. coli,* and proteins encoded by the aforementioned nucleotide sequences were expressed. These were subjected to functional screenings by assays on the xylan degradation activity. In the end, thermostable xylanases having xylanase activity (hereinafter, may be referred to as "AR19G-10-6") were obtained from these ORFs. The amino acid sequence of AR19G-10-6 and the nucleotide sequence encoding the amino acid sequence of AR19G-10-6 are represented by SEQ ID NO: 1 and SEQ ID NO: 2, respectively.

As shown in the section <9> of Example 1 described later, while exhibiting high hydrolysis activity for xylan, AR19G-10-6 exhibited almost no degradation activity for phosphoric acid swollen Avicel (PSA), Avicel which is a crystalline cellulose, carboxymethyl cellulose (CMC), lichenan composed of β-1,3- and β-1,4 glucans, laminarin composed of β-1,3- and β-1,6 glucans, PNPX (p-nitrophenyl-β-D-xylopyranoside) and PNPG (p-nitrophenyl-β-D-glucopyranoside).

It should be noted that in the present specification, the term "xylanase activity" refers to a hydrolytic activity towards xylan as a substrate. In addition, in the present specification, the expression "having an activity" refers to an action on at least one substrate and means that a significant difference occurs in the hydrolyzed amount of reducing ends of the substrate or the color reaction as compared to the negative control. Therefore, the expression "having xylanase activity" refers to an action on at least xylan and means that a significant difference occurs in the hydrolyzed amount of reducing ends of the substrate or the color reaction as compared to the negative control.

In addition, as another aspect, the expression "having xylanase activity" means having 6.0U / mg or more hydrolytic activity towards a least xylan under condition of a pH of 6.0 and in a temperature range of 40 to 99°C.

Further, as another aspect, the expression "having xylanase activity" means having 2.3U / mg or more hydrolytic activity towards at least xylan under condition of a temperature of 90°C and in a pH range of 4.0 to 8.0.

When the amino acid sequence of AR19G-10-6 was searched against publicly known amino acid sequence databases, the amino acid sequence showing the highest sequence identity was the amino acid sequence (SEQ ID NO: 6) of a xylanase (Genbank: AEY92972.1) of *Streptomyces hygroscopicus subsp. jinggangensis* 5008 belonging to the phylum Actinobacteria which belonged to the GH10 family, with the sequence identity (homology) thereof of 51% in full length and 56% in the xylanase catalytic domain. From the substrate specificity and the sequence identity of the amino acid sequence with that of the already known proteins, it is clear that AR19G-10-6 is a novel xylanase belonging to the GH10 family.

AR19G-10-6 has xylanase activity at least under conditions of a temperature of 90°C and a pH of 6.0. In fact, as shown in the section <10> in Example 1 described later, AR19G-10-6 exhibits xylanase activity within a temperature range from 40 to 99°C. More specifically, the xylanase activity of AR19G-10-6 increases as the temperature increases in the range from 40 to 90°C under condition of a pH of 6.0, and a large reduction in the activity cannot be seen even when the temperature exceeds 90°C. Not less than 80% of the activity in the optimum temperature was maintained even at a temperature of 99°C.

Generally, in a protein having some kind of bioactivity, one or two or more amino acids can be deleted, substituted, or added, without deteriorating the bioactivity. In other words, also in AR19G-10-6, one or two or more amino acids can be deleted, substituted, or added, without causing loss of glycoside hydrolytic activity, including xylanase activity.

That is, the thermostable xylanase according to the present invention is a thermostable glycoside hydrolase having a xylanase catalytic domain which includes any one of the following (A) to (C).
(A) A polypeptide including an amino acid sequence represented by SEQ ID NO: 1 (that is, AR19G-10-6);
(B) a polypeptide including an amino acid sequence in which at least one amino acid is deleted, substituted, or added in the amino acid sequence represented by SEQ ID NO: 1, as well as having xylanase activity at least under conditions of a temperature of 90°C and a pH of 6.0; or
(C) a polypeptide including an amino acid sequence having at least 75% sequence identity with the amino acid sequence represented by SEQ ID NO: 1, as well as having xylanase activity at least under conditions of a temperature of 90°C and a pH of 6.0.

In the present description, a "polypeptide in which an amino acid is deleted" means that a portion of the amino acids which constitute the polypeptide is missing (that is, removed).

In the present description, a "polypeptide in which an amino acid is substituted" means that an amino acid which constitutes the polypeptide is replaced with a different amino acid.

In the present description, a "polypeptide in which an amino acid is added" means that a new amino acid is inserted within the polypeptide.

In the aforementioned polypeptide of (B), the number of amino acids to be deleted, substituted, or added in the amino acid sequence represented by SEQ ID NO: 1 is preferably from 1 to 20, more preferably from 1 to 10, and still more preferably from 1 to 5.

In the aforementioned polypeptide of (C), the sequence identity with the amino acid sequence represented by SEQ ID NO: 1 is not particularly limited as long as it is 75% or greater but less than 100%, although it is preferable to be 80% or greater but less than 100%, more preferably 85% or greater but less than 100%, still more preferably 90% or greater but less than 100%, still more preferably 95% or greater but less than 100%, and particularly preferably 98% or greater but less than 100%.

It should be noted that the sequence identity (homology) between a pair of amino acid sequences is obtained such that: two amino acid sequences are juxtaposed while having gaps in some parts accounting for insertion and deletion so that the largest numbers of corresponding amino acids can be matched, and the sequence identity is deemed to be the proportion of the matched amino acids relative to the whole amino acid sequences excluding the gaps, in the resulting alignment. The sequence identity between a pair of amino acid sequences can be obtained by using a variety of homology search software publicly known in the art. The sequence identity value between amino acid sequences in the present invention is obtained by calculation on the basis of an alignment obtained from a publicly known homology search software BLASTP.

The aforementioned polypeptides of (B) and (C) may be those that are artificially designed, or may also be homologues of AR19G-10-6 and the like, or partial proteins thereof.

The aforementioned polypeptides of (A) to (C) may be respectively synthesized in a chemical manner based on the amino acid sequence, or may also be produced by a protein expression system using the polynucleotide according to the present invention that will be described later. In addition, the aforementioned polypeptides of (B) and (C) can also be respectively synthesized artificially based on a polypeptide including the amino acid sequence represented by SEQ ID NO: 1, by using a genetic recombination technique to introduce amino acid mutation(s).

The aforementioned polypeptides of (A) to (C) have xylanase activity at least under conditions of a temperature of 90°C and a pH of 6.0. For this reason, a thermostable xylanase can be obtained by having any one of the aforementioned polypeptides of (A) to (C) as the xylanase catalytic domain.

The thermostable xylanase according to the present invention uses xylan as a substrate. The aforementioned thermostable xylanase may further use another type of β-glucan or oligosaccharide other than xylan as a substrate. Examples of those that can be used as a substrate by the thermostable xylanase according to the present invention include PNPX, PNPG, p-nitrophenyl-α-L-arabinofuranoside, p-nitrophenyl-α-L-arabinopyranoside, p-nitrophenyl-β-L-arabinopyranoside, p-nitrophenyl-β-D-mannopyranoside, p-nitrophenyl-α-D-galactopyranoside, p-nitrophenyl-β-D-galactopyranoside; a glucan composed of β-1,3 bonds and β-1,4 bonds such as lichenan; a glucan composed of β-1,4 bonds including a crystalline cellulose, such as Avicel, a bacterial microcrystalline cellulose (BMCC) and a filter paper, a non-crystalline cellulose such as phosphoric acid swollen Avicel (PSA), and CMC; an oligosaccharide composed of β-1,4 bonds including such as cellobiose; a glucan composed of β-1,3 bonds and β-1,6 bonds such as laminarin; a glucan composed of β-1,3 bonds; a glucan composed of β-1,6 bonds and an oligosaccharide composed of β-1,6 bonds such as gentiobiose.

The thermostable xylanase according to the present invention preferably exhibits xylanase activity at least under condition of a pH of 6.0 within a temperature range from 80 to 95°C, more preferably within a temperature range from 70 to 99°C, still more preferably within a temperature range from 60 to 99°C and still more preferably within a temperature range from 50 to 100°C. The optimum temperature for the thermostable xylanase according to the present invention is preferably within the range from 80 to 99°C and more preferably within the range from 85 to 95°C.

Although the optimum pH of the thermostable xylanase according to the present invention varies depending on the reaction temperature, it is within a pH range from 5.0 to 7.0. As the thermostable xylanase according to the present invention, those exhibiting xylanase activity at least within the pH range of 5.0 to 8.0 are preferred, and those exhibiting xylanase activity within the pH range of 4 to 8.0 are more preferred.

The thermostable xylanase according to the present invention may also have a glycoside hydrolytic activity other than the xylanase activity. Examples of other glycoside hydrolytic activities include an endoglucanase activity, β-xylosidase activity, β-glucosidase activity and cellobiohydrolase activity.

The thermostable xylanase according to the present invention may be an enzyme solely consisting of a xylanase catalytic domain which includes any one of the aforementioned polypeptides of (A) to (C), or may further include other domains. Examples of other domains include a domain other than the enzyme catalytic domain present in the known xylanases. For example, the thermostable xylanase according to the present invention also includes enzymes obtained by substituting an enzyme catalytic domain in a publicly known xylanase with the aforementioned polypeptides of (A) to (C).

In addition, the thermostable xylanase according to the present invention may also have a signal peptide allowed to migrate to a specific region within a cell to be localized, or a signal peptide secreted to the outside of the cell, at the N-terminus or the C-terminus. Such a signal peptide can be exemplified by an apoplastic transport signal peptide, an endoplasmic reticulum retention signal peptide, a nuclear transport signal peptide, a secretory signal peptide, or the like. The endoplasmic reticulum retention signal peptide can be exemplified by, for example, a signal peptide including a HDEL amino acid sequence, or the like. In those cases where the thermostable xylanase according to the present invention has a signal peptide at the N-terminus or the C-terminus, the thermostable xylanase expressed in a transformant can be secreted outside the cell or localized in the endoplasmic reticulum and the like inside the cell.

In addition, the thermostable xylanase according to the present invention may also be added with, for example, various types of tags at the N-terminus or the C-terminus, so as to enable easy and convenient purification in a case of the production using an expression system. Regarding such a tag, it is possible to use a tag for usual use in the expression or purification of a recombinant protein, such as a His tag, a HA (hemagglutinin) tag, a Myc tag, and a Flag tag.

### [Polynucleotide that encodes thermostable xylanase]

A polynucleotide according to the present invention encodes the thermostable xylanase according to the present invention. The aforementioned thermostable xylanase can be produced by using the expression system of a host made by introducing an expression vector incorporated with the polynucleotide into the host.

More specifically, the polynucleotide according to the present invention is a polynucleotide having a region that encodes a xylanase catalytic domain which includes any one of the following nucleotide sequences (a) to (e).
(a) A nucleotide sequence that encodes a polypeptide including the amino acid sequence represented by SEQ ID NO: 1.
(b) A nucleotide sequence that encodes a polypeptide including an amino acid sequence in which at least one amino acid is deleted, substituted, or added in the amino acid sequence represented by SEQ ID NO: 1, as well as having xylanase activity at least under conditions of a temperature of 90°C and a pH of 6.0.
(c) A nucleotide sequence that encodes a polypeptide including an amino acid sequence having at least 75% sequence identity with the amino acid sequence represented by SEQ ID NO: 1, as well as having xylanase activity at least under conditions of a temperature of 90°C and a pH of 6.0.
(d) A nucleotide sequence having at least 75% sequence identity with the nucleotide sequence represented by SEQ ID NO: 2, as well as encoding a polypeptide having xylanase activity at least under conditions of a temperature of 90°C and a pH of 6.0.
(e) A nucleotide sequence of a polynucleotide which hybridizes with a polynucleotide including a nucleotide sequence represented by SEQ ID NO: 2 under a stringent condition, as well as being a nucleotide sequence that encodes a polypeptide having xylanase activity at least under conditions of a temperature of 90°C and a pH of 6.0.

It should be noted that in the present description of this application, a "polynucleotide in which a nucleotide is deleted" means that a portion of the nucleotides which constitute the polynucleotide is missing (that is, removed).

In the present description of this application, a "polynucleotide in which a nucleotide is substituted" means that a nucleotide which constitutes the polynucleotide is replaced with a different nucleotide.

In the present description of this application, a "polynucleotide in which a nucleotide is added" means that a new nucleotide is inserted within the polynucleotide.

In the present description of this application, the term "under a stringent condition" can be exemplified by the method described in Molecular Cloning - A Laboratory Manual Third Edition (Sambrook et al., Cold Spring Harbor Laboratory Press). The example thereof includes a condition in which hybridization is performed by incubation in a hybridization buffer including 6 × SSC (composition of 20 × SSC: 3M sodium chloride, 0.3M citric acid solution, and pH7.0), 5 × Denhardt's solution (composition of 100 × Denhardt's solution: 2 mass% bovine serum albumin, 2 mass% Ficoll, 2 mass% polyvinylpyrrolidone), 0.5 mass% SDS, 0.1 mg/mL salmon sperm DNA, and 50% formamide, at a temperature of 42 to 70°C for several hours to overnight. The washing buffer for use in the washing after the incubation is preferably 1 × SSC solution containing 0.1 mass% SDS, and more preferably 0.1 × SSC solution containing 0.1 mass% SDS.

In the aforementioned nucleotide sequences of (a) to (e), it is preferable to select a degenerate codon having high frequency of usage in the host. For example, the aforementioned nucleotide sequence of (a) may be either the nucleotide sequence represented by SEQ ID NO: 2, or a nucleotide sequence altered to have a codon having high frequency of usage in the host without changing the amino acid sequence to be encoded by the nucleotide sequence represented by SEQ ID NO: 2. Note that, these codons can be altered by a publicly known gene sequence modification technique or artificial gene synthesis.

The polynucleotide including the nucleotide sequence represented by SEQ ID NO: 2 may be synthesized in a chemical manner based on the nucleotide sequence information, or may be obtained as a full length of a gene that encodes AR19G-10-6 (may be referred to as "AR19G-10-6 gene" or "gene clone AR19G-10-6") or a partial region thereof including the xylanase catalytic domain (that is, a region encoding a partial region composed of 312 amino acid residues from leucine (L) at position 35 to methionine (M) at position 346 in SEQ ID NO: 1) from the natural world by using a genetic recombination technique. The full length of the AR19G-10-6 gene or the partial region thereof can be obtained by, for example, collecting a sample containing microorganisms from the natural world, and conducting PCR using the genomic DNA recovered from the sample as a template, with a forward primer and a reverse primer designed on the basis of the nucleotide sequence represented by SEQ ID NO: 2 by a conventional method. The cDNA synthesized by a reverse transcription reaction using mRNA recovered from the sample as a template may also be used as a template. Note that, it is preferable that the sample for recovering the nucleic acid serving as a template is a sample collected from a high temperature environment such as hot spring soil.

In the aforementioned nucleotide sequence of (d), the sequence identity with the nucleotide sequence represented by SEQ ID NO: 2 is not particularly limited as long as it is 75% or greater but less than 100%, although it is preferable to be 80% or greater but less than 100%, more preferably 85% or greater but less than 100%, still more preferably 90% or greater but less than 100%, still more preferably 95% or greater but less than 100%, and particularly preferably 98% or greater but less than 100%.

Note that, the sequence identity (homology) between a pair of nucleotide sequences is obtained such that: two nucleotide sequences are juxtaposed while having gaps in some parts accounting for insertion and deletion so that the largest numbers of corresponding nucleotides can be matched, and the sequence identity is deemed to be the proportion of the matched nucleotides relative to the whole nucleotide sequences excluding gaps, in the resulting alignment. The sequence identity between a pair of nucleotide sequences can be obtained by using a variety of homology search software publicly known in the art. The sequence identity value between nucleotide sequences in the present invention is obtained by calculation on the basis of an alignment obtained from a publicly known homology search software BLASTN.

For example, the polynucleotide including the aforementioned nucleotide sequence of (b), (c), or (d) can be respectively synthesized artificially by deleting, substituting, or adding one or two or more nucleotides in a polynucleotide including the nucleotide sequence represented by SEQ ID NO: 2. In addition, the aforementioned nucleotide sequence of (b), (c), or (d) may also be a full length sequence of a homologous gene of the AR19G-10-6 gene or a partial sequence thereof. The homologous gene of the AR19G-10-6 gene can be obtained by a genetic recombination technique for use in obtaining a homologous gene of a gene whose nucleotide sequence has been already known.

The polynucleotide according to the present invention may have only the region that encodes the xylanase catalytic domain, or may also have a region that encodes a cellulose-binding module, a linker sequence, various types of signal peptides, various types of tags, or the like, in addition to the aforementioned region.

### [Expression vector]

The expression vector according to the present invention is incorporated with the aforementioned polynucleotide according to the present invention, and is able to express a polypeptide having xylanase activity at least under conditions of a temperature of 90°C and a pH of 6.0 in a host cell. That is, it is an expression vector which is incorporated with the aforementioned polynucleotide according to the present invention in a state where the aforementioned thermostable xylanase according to the present invention can be expressed. More specifically, it is necessary for the expression vector to be incorporated with an expression cassette including, from the upstream, DNA having a promoter sequence, the aforementioned polynucleotide according to the present invention, and DNA having a terminator sequence. It should be noted that the incorporation of the polynucleotide into the expression vector can be performed by using a well-known genetic recombination technique by using the well-known genetic recombination techniques. It is also possible to use a commercially available expression vector preparation kit for the incorporation of the polynucleotide into the expression vector.

In the description of the present invention, an "expression vector" is a vector including, from upstream, DNA having a promoter sequence, DNA having a sequence for incorporating foreign DNA, and DNA having a terminator sequence.

The expression vector may be a vector to be introduced into a prokaryotic cell such as *E. coli,* or to be introduced into a eukaryotic cell such as a yeast, a filamentous fungus, a cultured insect cell, a cultured mammalian cell, or a plant cell. Regarding such an expression vector, an arbitrary expression vector for usual use can be adopted corresponding to the respective host.

It is preferable that the expression vector according to the present invention is an expression vector incorporated with not only the aforementioned polynucleotide according to the present invention but also a drug resistance gene or the like. This is because it makes it easy to screen host cells transformed by the expression vector and untransformed host cells.

The drug resistance gene can be exemplified by a kanamycin resistance gene, a hygromycin resistance gene, a bialaphos resistance gene, or the like.

### [Transformant]

The transformant according to the present invention is introduced with the above-mentioned expression vector according to the present invention. In the transformant, the above-mentioned thermostable xylanase according to the present invention can be expressed. The host to introduce the expression vector may be a prokaryotic cell such as *E. coli* or a eukaryotic cell such as a yeast, a filamentous fungus, a cultured insect cell, a cultured mammalian cell, or a plant cell. That is, the transformant according to the present invention is *E. coli,* a yeast, a filamentous fungus, an cultured insect cell, a cultured mammalian cell, a plant cell, or the like which is introduced with the expression vector according to the present invention.

By culturing a transformant of *E. coli,* the thermostable xylanase according to the present invention can be produced more easily and conveniently with high yield. On the other hand, because proteins are hydrolyzed in eukaryotic cells, a thermostable xylanase which is more thermostable can be produced by using a transformant of a eukaryotic cell rather than by using a transformant of a prokaryotic cell.

The method to produce the transformant using the expression vector is not particularly limited, and a method for usual use in the production of transformants can be conducted. The concerned method can be exemplified by a heat shock method, an Agrobacterium-mediated method, a particle gun method, an electroporation method, a PEG (polyethylene glycol) method, and the like. Of these, if the host is a plant cell, a particle gun method or an Agrobacterium-mediated method is preferred.

If a prokaryotic cell, a yeast, a filamentous fungus, a cultured insect cell, a cultured mammalian cell, or the like is used as a host, the obtained transformant is generally able to be cultured by a usual method in the same manner as that of the untransformed host.

### [Method for producing a thermostable xylanase]

The method for producing a thermostable xylanase according to the present invention is a method to produce a thermostable xylanase in the aforementioned transformant according to the present invention. When culturing a transformant produced by using the expression vector incorporated with the aforementioned polynucleotide according to the present invention on the downstream of a promoter which has no ability to regulate the timing of the expression or the like, in the transformant, the thermostable xylanase according to the present invention is expressed constitutively. On the other hand, for the transformant produced by using a so-called expression inducible promoter to induce the expression by means of a specific compound, temperature condition, or the like, the thermostable xylanase is expressed in the concerned transformant by culturing the transformant and conducting an induction treatment suitable for the respective expression-inducing condition.

The thermostable xylanase produced by the transformant may be used in a state of being retained in the transformant, or may be extracted and purified from the transformant.

The method to extract or purify the thermostable xylanase from the transformant is not particularly limited as long as the method does not deteriorate the activity of the thermostable xylanase, and the extraction can be done by a method for usual use in the extraction of a polypeptide from cells or biological tissues. The method can be exemplified by a method in which the transformant is immersed in an appropriate extraction buffer to extract the thermostable xylanase, and thereafter the liquid extract and the solid residue are separated. The extraction buffer preferably contains a solubilizing agent such as a surfactant. If the transformant is a plant, the transformant may be previously shredded or crushed before immersing in an extraction buffer.

Moreover, as the method for separating the liquid extract and the solid residue, for example, a publicly known solid-liquid separation treatment such as a filtration method, a compression filtration method, or a centrifugation treatment method may be used, or the transformant immersed in an extraction buffer may be squeezed. The thermostable xylanase in the liquid extract can be purified by using a publicly known purification method such as a salting-out method, an ultrafiltration method, or a chromatography method.

If the thermostable xylanase according to the present invention is expressed while the secretory signal peptide is held in the transformant, a solution containing the thermostable xylanase can be easily and conveniently obtained by culturing the transformant and thereafter recovering a culture liquid supernatant made by removal of the transformant from the obtained culture product. Moreover, if the thermostable xylanase according to the present invention has a tag such as a His tag, the thermostable xylanase in a liquid extract or in a culture supernatant can be easily and conveniently purified by an affinity chromatography method using the tag.

In other words, the method for producing a thermostable xylanase according to the present invention includes the production of a thermostable xylanase within the transformant according to the present invention, and, according to need, the extraction and purification of the thermostable xylanase from the transformant.

### [Glycoside hydrolase mixture]

The glycoside hydrolase mixture according to the present invention can also be used as a glycoside hydrolase mixture containing the aforementioned thermostable xylanase according to the present invention, or a thermostable xylanase produced by the aforementioned method for producing a thermostable xylanase according to the present invention, and at least one or more types of other glycoside hydrolases. The thermostable xylanase produced by the aforementioned method for producing a thermostable xylanase according to the present invention may be in a state of being included in the transformant, or may be extracted or purified from the transformant. By using the thermostable xylanase according to the present invention as a mixture with other glycoside hydrolases in the reaction to hydrolyze polysaccharides, persistent lignocelluloses can be more efficiently degraded.

The other glycoside hydrolase than the aforementioned thermostable xylanase to be contained in the glycoside hydrolase mixture is not particularly limited as long as it is an enzyme having a hydrolytic activity of lignocellulose. The other glycoside hydrolase than the aforementioned thermostable xylanase to be contained in the glycoside hydrolase mixture can be exemplified by hemicellulases such as β-xylosidase, cellobiohydrolase, β-glucosidase, endoglucanase, or the like. In addition to the thermostable xylanase, the glycoside hydrolase mixture according to the present invention is preferably a mixture containing at least one of glycoside hydrolases (i.e., a hemicellulase or an endoglucanase), and is more preferably a mixture containing both glycoside hydrolases (i.e., a hemicellulase and an endoglucanase). Among them, a mixture containing at least one of glycoside hydrolases selected from the group consisting of xylanase, β-xylosidase, cellobiohydrolase, and endoglucanase is preferred; and a mixture containing all of glycoside hydrolases (i.e., xylanase, β-xylosidase, cellobiohydrolase, and endoglucanase) are more preferred.

The other glycoside hydrolase to be contained in the glycoside hydrolase mixture is preferably a thermostable glycoside hydrolase having glycoside hydrolase activity at least at a temperature of 85°C, and more preferably a thermostable glycoside hydrolase having glycoside hydrolase activity at a temperature of 70 to 90°C. When all the enzymes contained in the glycoside hydrolase mixture are thermostable (for example, the optimum temperature of the enzyme activity or the thermal denaturation temperature of the enzyme protein is 70°C or higher), the reaction to degrade lignocellulose with the glycoside hydrolase mixture can be efficiently conducted under a high temperature condition. That is, if the glycoside hydrolase mixture contains only thermostable glycoside hydrolases, it becomes possible, by using the glycoside hydrolase mixture for a lignocellulose hydrolysis process, to conduct the lignocellulose hydrolysis reaction under a high temperature environment with a hydrolysis temperature of 70 to 90°C (high temperature hydrolysis). With this high temperature hydrolysis, the amount of enzymes and the time for hydrolysis can be remarkably reduced, and the cost for hydrolysis can be

largely cut out.

### [Method for producing a lignocellulose degradation product]

The method for producing a lignocellulose degradation product according to the present invention is a method to obtain a lignocellulose degradation product by hydrolyzing hemicellulose to produce oligosaccharides with the thermostable xylanase according to the present invention. More specifically, a hemicellulose degradation product is produced by bringing hemicellulose, more specifically, a material composed of lignocellulose containing hemicellulose including xylan, into contact with the thermostable xylanase according to the present invention, the transformant according to the present invention, the thermostable xylanase produced by the method for producing a thermostable xylanase according to the present invention, or the glycoside hydrolase mixture according to the present invention.

As hemicellulose degradation product, for example, xylose, xylooligosaccharide and the like can be mentioned.

Hemicellulose, more specifically, the material composed of lignocellulose containing hemicellulose including xylan, is not particularly limited as long as it contains hemicellulose, more specifically, xylan. As the above material, for example, biomass such as weeds and agricultural waste, waste paper and the like can be mentioned. The above material is preferably subjected to a physical treatment such as crushing or shredding, a chemical treatment with an acid, alkali, or the like, or an immersion or dissolution treatment in an appropriate buffer or the like, prior to being brought into contact with the thermostable xylanase according to the present invention.

The reaction condition of the hydrolysis reaction of hemicellulose, more specifically, xylan, by means of the thermostable xylanase according to the present invention may suffice if the condition allows the thermostable xylanase to exhibit xylanase activity. For example, it is preferable to conduct the reaction at a temperature of 60 to 99°C and a pH of 4.0 to 8.0, more preferable to conduct the reaction at a temperature of 70 to 99°C and a pH of 5.0 to 7.0, and still more preferable to conduct the reaction at a temperature of 80 to 99°C and a pH of 5.0 to 7.0. The reaction time of the hydrolysis reaction is appropriately adjusted in consideration of the type, the method of pretreatment, the amount, or the like, of the material composed of lignocellulose containing hemicellulose, more specifically, hemicellulose including xylan, to be supplied to the hydrolysis. For example, the hydrolysis reaction can be carried out in a reaction time of 10 minutes to 100 hours, and 1 to 100 hours when degrading the cellulose-based biomass.

For the hydrolysis reaction of lignocellulose, it is also preferable to use at least one or more types of other glycoside hydrolases simultaneously or separately, in addition to the thermostable xylanase according to the present invention. The other glycoside hydrolase may be the same as the glycoside hydrolase that can be contained in the aforementioned glycoside hydrolase mixture, and it is preferable to be a thermostable glycoside hydrolase having a glycoside hydrolase activity at least at a temperature of 85°C, and preferably at least at a temperature of 70 to 90°C. In addition, one aspect of the method for producing a lignocellulose degradation product is the use of the thermostable xylanase according to the present invention, the transformant according to the present invention, or a thermostable xylanase produced by the method for producing a thermostable xylanase according to the present invention, and another aspect is the use of the aforementioned glycoside hydrolase mixture.

### [Examples]

Next is a more detailed description of the present invention with reference to Examples. However, the present invention is not to be limited to the following Examples.

### [Example 1] Cloning of novel thermostable xylanase from hot spring soil

### <1> DNA extraction from hot spring soil and Whole Genome Sequencing (WGS)

With the purpose of searching for genes of novel thermostable xylanase exhibiting activity at a temperature of 70 to 99°C, soil DNA was collected from neutral to weakly alkaline hot springs and subjected to nucleotide sequencing of the metagenomic DNA of the microbiota constituting the soil.

As the soil sample from neutral to weakly alkaline hot springs, hot spring water containing soil, clay, and biomat was collected from five sampling points having gushing high temperature outdoor hot springs in three areas in Japan (metagenomic DNA samples N2, AR19, AR15, OJ1, and H1). These hot spring soil samples were within a range of temperature from 58 to 78°C and a pH of 7.2 to 8 at the time of the collection.

DNA was extracted from 10g each of the collected hot spring soil samples by using the DNA extraction kit (ISOIL Large for Beads ver.2, manufactured by NIPPON GENE Co., Ltd.). 5 µg of the extracted DNA was subjected to shotgun sequencing of the metagenomic DNA by using the sequencer GS FLX Titanium 454 manufactured by Roche Diagnostics K.K.

The metagenomic DNA sequencing was carried out using the hot spring soil sample AR19. By so doing, a data set of the whole genome sequence (WGS) with an average read length of 396bp, a total read number of 2,766,332, and a total quantity of sequenced genomes of 1,106,243,280bp, was obtained.

### <2> Assembling and statistics of hot spring metagenomic data

The output from the Roche 454 (sff file) was rebasecalled with the PyroBayes (Quinlan et al., Nature Methods, 2008, vol.5, p.179-81.), by which sequence files and quality files in FASTA format were obtained. After clipping their ends to improve the quality, the obtained sequence reads were assembled with use of the assembly software, Newbler version 2.3 of 454 Life Sciences. The assembling was performed by setting "minimum acceptable overlap match (mi) = 0.9", and "option:-large (for large or complex genomes, speeds up assembly, but reduces accuracy.)".

The total length of the contigs that have been assembled into 100 bp or longer was 104,096,316 bp in total. This data set was used for the xylanase enzyme gene analysis. Out of the total read number of 2,766,332 reads, 2,040,651 reads were assembled into 1,027 bp or longer contigs in average (101,372 contigs in total). Of these, the longest contig length was 187,970 bp.

### <3> Prediction of open reading frames (ORFs) of xylanase

The sequences of EC numbers of 3.2.1.4 (cellulase), 3.2.1.21 (β-glucosidase), 3.2.1.37 (β-xylosidase), 3.2.1.91 (cellulose 1,4-β-cellobiosidase), and 3.2.1.8 (endo 1,4-β-xylanase) were downloaded from the UniProtdatbase (http://www.uniprot.org/) (the date of access: 2011/12/9), and the proteome local database of these glycoside hydrolase genes was constructed. Using the annotation software Orphelia (Hoff et al., Nucleic Acids Research, 2009, 37 (Web Server issue: W101-W105), gene regions (= open reading frames) were predicted from the contig sequences obtained from the above-mentioned process <2> (Orphelia option: default (model = Net700, maxoverlap = 60). In order to extract the glycoside hydrolase gene from the predicted ORF, the above-mentioned local database using BLASTP (blastall ver. 2.2.18) was referred to. Optional conditions of BLASTP were set such that: "Filter query sequence = false", "Expectation value (E) <1e-20" [hereunder, the default values: Cost to open a gap = -1, Cost to extended gap = -1, X dropoff value for gapped alignment = 0, Threshold for extending hits = 0, and Word size = default], and the hit ORF sequences were collected as glycoside hydrolase genes. The collected nucleotide sequences included the genes of glycoside hydrolases such as cellulases, endohemicellulases, and debranching enzymes.

### <4> Classification of genes into glycoside hydrolase (GH) families

The nucleotide sequences that had been collected in the above-mentioned process <3> were subjected to functional classification, with reference to the protein functional region sequence database of pfam HMMs (Pfam version 23.0 and HMMER v2.3; Finn et al., Nucleic Acids Research Database, 2010, Issue 38, p.D211-222). More specifically, the glycoside hydrolase (GH) families were determined for each of the nucleotide sequences that had been collected in the above-mentioned process <3> by the homology with the Pfam domain database by using the protein motif search program HMMER (Durbin et al., "The theory behind profile HMMs. Biological sequence analysis: probabilistic models of proteins and nucleic acids", 1998, Cambridge University Press.; hmmpfam (Ver.2.3.2), E-value cutoff <1e⁻⁵; Database = Pfam_fs (models that can be used to find fragments of the represented domains in a sequence.)). It should be noted that those that covered 70% or more of the sequence of GH catalytic domain were counted as enzyme genes belonging to the respective families.

Through the homology search by BLASTP and domain search by HMMER by using the sequence data of metagenome AR19, 33 ORFs (20 full-length ORFs and 13 incomplete ORFs) were predicted as xylanase genes. The GH family classification of these ORFs is shown in Table 1. As shown in Table 1, seven full-length ORFs of xylanase genes belonging to the GH family 10 and one full-length ORF of xylanase gene belonging to the GH family 11 were obtained from the metagenome AR19. Primers were designed for all of these ORFs having been predicted as xylanase genes, and these genes were cloned from the hot spring soil metagenomic DNA by PCR. As a result, a xylanase gene was isolated from the open reading frame AR19G-10 belonging to the GH family 10 and having the sequence of a xylanase gene.

**[Table 1]**

| AR19 metagenome | GH family classification of xylanase genes | | | |
|---|---|---|---|---|
| | GH10 | GH11 | Other GH families | Total |
| Full-length ORFs | 7 | 1 | 12 | 20 |
| Incomplete ORFs | 10 | 0 | 3 | 13 |
| Total number of ORFs | 17 | 1 | 15 | 33 |

### <5> Open reading frame AR19G-10

The open reading frame AR19G-10 encoded a polypeptide (SEQ ID NO: 1) including 357 amino acid residues and was a full-length sequence (SEQ ID NO: 2), such that the polypeptide started from methionine which was an amino acid residue at position 1, and the 3' end of the nucleotide sequence encoding the polypeptide ended with a termination codon. According to analysis using the signal sequence prediction software SignalP 4.1, in the polypeptide encoded by the open reading frame AR19G-10, 28 amino acid residues from methionine (M) at position 1 to serine (S) at position 28 was a secretion signal. In addition, from the sequence homology of motifs, in the polypeptide encoded by the open reading frame AR19G-10, it was predicted that 312 amino acid residues from leucine (L) at position 35 to methionine (M) at position 346 encoded the catalytic domain of the Glycoside hydrolase family 10. The known amino acid sequence that showed the highest sequence identity with the amino acid sequence encoded by the aforementioned ORF was a GH10 xylanase of *Streptomyces hygroscopicus subsp. jinggangensis* 5008 (Genbank: AEY92972.1) of the phylum of the Actinobacteria. Since the homology between the two amino acid sequences that was calculated by the ClustalW algorithm was 51% for the total length and 56% for the GH10 catalytic domain, the aforementioned ORF was verified as a novel sequence.

### <6> Gene cloning

PCR was conducted using a hot spring soil DNA that had been amplified by the genomic DNA amplification kit (GenomiPhi V2 DNA Amplification Kit, manufactured by GE Healthcare) as a template, and by using a forward primer composed of the nucleotide sequence represented by SEQ ID NO: 5 (5'-CACCATGAAGCTCTATCGGTACGGCTTG-3': 4 nucleotides (CACC) were added to the 5'-end side of the nucleotide sequence represented by SEQ ID NO: 3. The nucleotides CACC added on the 5' side is a sequence for insertion into a vector) and a reverse primer composed of the nucleotide sequence represented by SEQ ID NO: 4 (5'-CTACCTCCCTTTGGCGGCTACATA-3'). The nucleotide sequence represented by SEQ ID NO: 3 is a nucleotide sequence which is homologous (identical) with a partial sequence including the nucleotides at position 1 to 24 of the nucleotide sequence represented by SEQ ID NO: 2. Moreover, the nucleotide sequence represented by SEQ ID NO: 4 is a nucleotide sequence which is complementary with a partial sequence including the nucleotides at position 1,051 to 1,074 of the nucleotide sequence represented by SEQ ID NO: 2.

The amplified PCR products were inserted in the pET101/D-TOPO vector of Champion pET Directional TOPO Expression Kits (manufactured by Life Technologies), and transformed into the One Shot TOP10 strain. Positive clones were selected by colony PCR, and then cultured in a LB liquid medium containing 100 mg/L ampicillin at a temperature of 37°C and 200 rpm for 17 to 20 hours, followed by the preparation of plasmids using the miniprep kit (Wizard plus SV Minipreps DNA Purification System, manufactured by Promega). The prepared plasmids were sequenced by using the 3730 DNAAnalyzer sequencer of Life Technologies.

Five gene clones AR19G-10-5, AR19G-10-6, AR19G-10-7, AR19G-10-11 and AR19G-10-21 were obtained from the open reading frame AR19G-10 by PCR cloning. The nucleotide sequence of the gene clone AR19G-10-6 which was a xylanase candidate gene was completely identical with that of the open reading frame AR19G-10 (SEQ ID NO: 2), and was encoding a polypeptide (AR19G-10-6) composed of 357 amino acid residues (SEQ ID NO: 1).

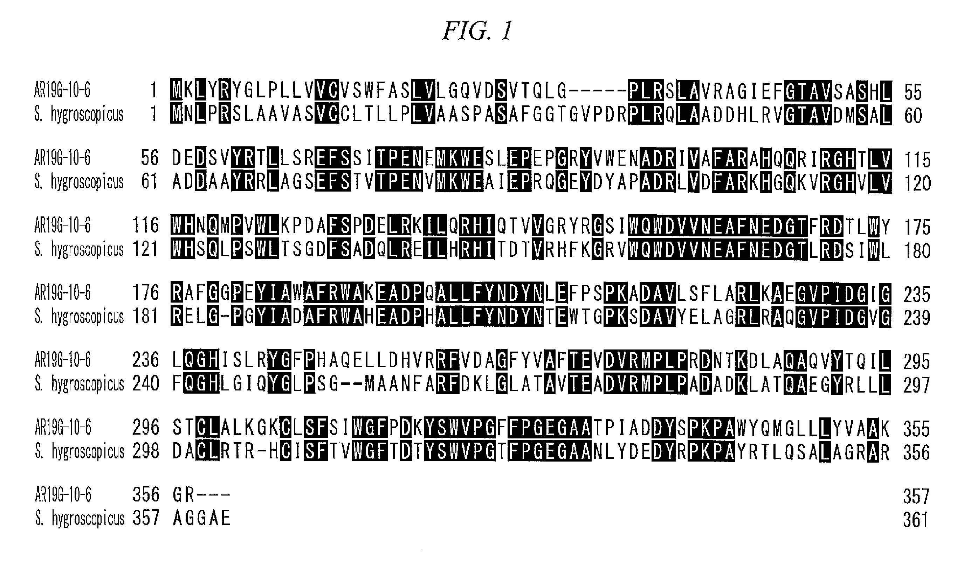

FIG. 1 shows an alignment of the amino acid sequence of the gene clone AR19G-10-6 and the amino acid sequence of a GH10 xylanase (SEQ ID NO: 6) of *Streptomyces hygroscopicus subsp. jinggangensis* 5008 belonging to the phylum Actinobacteria. In FIG. 1, the black/white inverted amino acids denote the same amino acid residues (identical) throughout all of these amino acid sequences, and the symbols "- " denote deletions (gaps).

### <7> Gene expression and purification of xylanase enzymatic protein

After the sequencing, the plasmids having the target gene were introduced in *E*. *coli* for protein expression by a heat shock method. The BL21 Star (DE3) strain furnished in the Champion pET Directional TOPO Expression Kits (manufactured by Life Technologies) was used as the competent cell for the transformation. *E. coli* having the target gene was inoculated in a LB medium containing 100 mg/L ampicillin and cultured to about OD600 = 0.2 to 0.8, which was then added with IPTG (isopropyl-β-D(-)-thiogalactopyranoside), and additionally cultured for 5 to 20 hours. By so doing, the expression induction of the target protein was carried out. After the culture, *E. coli* was collected by centrifugation, to which 50 mM Tris-HCl buffer (pH8.0) of 1/10-fold volume of the culture liquid was added and suspended. Thereafter, 5 minutes disrupting and 5 minutes halting processes were repeated 7 to 8 times by using an ultrasonic disruption apparatus, Astrason 3000 (manufactured by Misonix, Inc.). By so doing, the crude extract of the gene recombinant *E. coli* containing the target protein was obtained. The crude extract of the gene recombinant *E. coli* was filtrated through a filter (pore diameter ϕ = 0.45 µm, manufactured by Millipore), and the yielded filtrate was used as a gene recombinant *E. coli* homogenate supernatant.

The gene recombinant *E. coli* homogenate supernatant was loaded onto an ionexchange column HiTrap Q HP (manufactured by GE Healthcare) equilibrated with 50 mM Tris-HCl buffer (pH8.0), by which proteins were fractionated with 0 to 50% concentration gradient with 50 mM Tris-HCl buffer (pH8.0) containing 1M NaCl using a middle-to-high pressure liquid chromatography system AKTA design (manufactured by GE Healthcare). The fractions exhibiting xylanase activity were all mixed and then subjected to solution exchange into 50 mM Tris-HCl buffer (pH8.0) containing 750 mM ammonium sulfate using a centrifugal ultrafiltration membrane VIVASPIN 20 (manufactured by Sartorius stedim). The fractions with xylanase activity after the solution exchange were loaded onto a hydrophobic interaction separation column HiTrap Phenyl HP (manufactured by GE Healthcare) equilibrated with the same solution, by which proteins were fractionated with 0 to 100% concentration gradient with 50 mM Tris-HCl buffer (pH8.0). The fractions exhibiting xylanase activity were all mixed and then concentrated by using the VIVASPIN 20 until the liquid volume reached to about 8 mL. The concentrated sample was loaded onto a gel filtration column Hiload 26/60 superdex 200 pg (manufactured by GE Healthcare) equilibrated with 50 mM Tris-HCl buffer (pH8.0) containing 150 mM NaCl, and fractionated by flowing the same buffer of 1 to 1.5 fold volume of the column volume at a flow rate of 2 to 3 mL/min. The fractions exhibiting xylanase activity were all mixed and then subjected to solution exchange into 50 mM Tris-HCl buffer (pH8.0) and concentrated. By so doing, a purified enzyme having the final concentration of about 1 mg/mL was obtained.

The gene recombinant *E. coli* homogenate supernatant and the purified enzyme were checked by SDS-PAGE (SDS-polyacrylamide gel electrophoresis) analysis. The SDS-PAGE of the gene recombinant *E. coli* homogenate supernatant and the purified enzyme was conducted by using the Mini-PROTEAN TGX Stain-Free Gel (manufactured by Bio-Rad Laboratories, Inc.). The electrophoresis samples prepared by respectively mixing the supernatant or the purified enzyme with Tris-SDS βME treatment solution (manufactured by Cosmo Bio Co., Ltd.) at 1:1 were treated at a temperature of 100°C for 10 minutes. Then, 10 µL of the gene recombinant *E. coli* homogenate supernatant and 1 µg of the purified enzyme per each sample were respectively electrophoresed. After the electrophoresis, the protein bands were visualized by CBB staining.

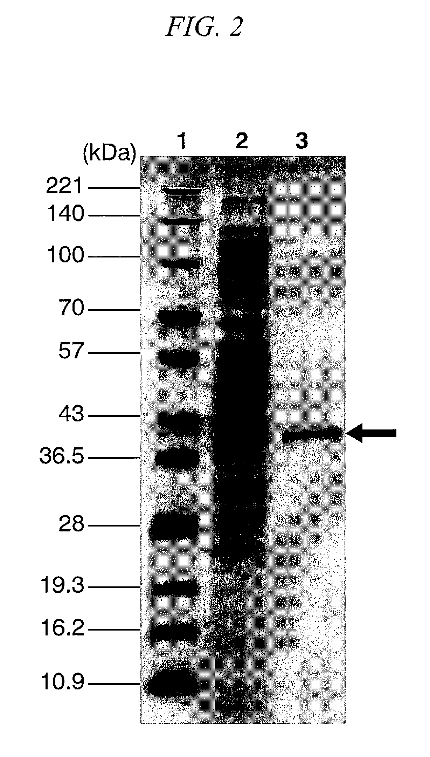

FIG. 2 shows the SDS-PAGE analysis result of the gene recombinant *E. coli* homogenate supernatant prepared from the transformed *E. coli* introduced with the AR19G-10-6 gene and the purified enzyme which was purified from the gene recombinant *E. coli* homogenate supernatant. The lane 1 is a molecular weight marker for proteins, and the lanes 2 and 3 show the electrophoresis patterns of the gene recombinant *E. coli* homogenate supernatant and purified enzyme, respectively. As a result, in the gene recombinant *E. coli* homogenate supernatant (lane 2), a strong band was observed near the molecular weight of 40.7 kDa predicted from the amino acid sequence (SEQ ID NO: 1), and in the purified enzyme (lane 3), a single band corresponding with the above band was observed (indicated by an arrow in the figure).

### <8> Measurement of xylanase activity (xylan hydrolysis activity)

Xylan (derived from beechwood, manufactured by Sigma) was used as a substrate in the measurement of the xylanase activity. A solution prepared by dissolving xylan in water and adjusting the concentration to be 1% by mass with respect to the total mass (hereinafter, may be referred to as a "1% by mass xylan aqueous solution") was used as a substrate solution. It should be noted that the 1% by mass xylan aqueous solution prepared by the above method was used as the xylan aqueous solution used for all the following experiments.

The xylanase activity of the enzyme protein (AR19G-10-6) encoded by the AR19G-10-6 gene was investigated. More specifically, a mixture solution including 100 µL of the 1% by mass xylan aqueous solution, 50 µL of 200 mM phosphoric acid buffer (pH6.0), either 50 µL of the gene recombinant *E. coli* homogenate supernatant obtained in the above-mentioned process <7> or 2 µL of the purified enzyme (0.1 mg/mL) diluted with 50 mM Tris-HCl buffer (pH8.0) and 48 µL of purified water was allowed to react at a temperature of 40 to 99°C for 10 minutes. In the reaction, stirring was conducted by using the Eppendorf's Thermomixer (1400 rpm) so as to avoid the precipitation of xylan. In all the measurements, a mixture solution prepared by adding 50 mM Tris-HCl buffer (pH8.0) instead of the gene recombinant *E. coli* homogenate supernatant or purified enzyme and reacting under the same conditions was used as the control lot. Moreover, the substrate solution and the enzyme (gene recombinant *E. coli* homogenate supernatant or purified enzyme) were respectively and separately kept at retained reaction temperatures for 5 minutes, and then mixed. This timing was set to the initiation of the reaction. After the completion of the reaction, the same volume of a 3,5-dinitrosalicylic acid reagent (DNS solution) was added to each mixture solution. The resulting mixtures were treated by heating at a temperature of 100°C for 5 minutes, cooled down on ice for 5 minutes, and then centrifuged at 17,400g for 5 minutes. By so doing, the supernatant was obtained. The absorbance at 540 nm was measured by using a spectrophotometer, and the amount of reduced sugar in the supernatant was calculated by using a calibration curve prepared with xylose. The amount of reduced sugar produced by the enzymatic hydrolysis was obtained by the difference from the control lot. The enzymatic activity for producing 1 µmol of reduced sugar per minute was s defined as 1U, and the value obtained by dividing it by the amount of protein was defined as the specific activity (U/mg).

As a result, the presence of xylanase activity was confirmed in both cases where the gene recombinant *E. coli* homogenate supernatant was used and where the purified enzyme was used.

### <9> Substrate specificity of AR19G-10-6

The hydrolysis activities for various cellulose substrates and hemicellulose substrates were investigated with the enzyme protein (AR19G-10-6) encoded by the AR19G-10-6 gene. In the measurement, a purified enzyme solution (0.1 mg/mL) prepared by diluting the purified enzyme (concentration of about 1 mg/mL) obtained from the above-mentioned process <7> with 50 mM Tris-HCl buffer (pH8.0) was used. In addition, as substrates, PSA, an Avicel powder (microcrystalline cellulose powder, manufactured by Merck), CMC (manufactured by Sigma), xylan (derived from beechwood, manufactured by Sigma), lichenan (manufactured by MP Biomedicals), laminarin (derived from Laminaria digitata, manufactured by Sigma), PNPX (manufactured by Sigma), and PNPG (manufactured by Sigma) were used.

PSA was prepared by once dissolving an Avicel powder (microcrystalline cellulose powder, manufactured by Merck) with a phosphoric acid solution, then precipitating it by adding sterile purified water, and thereafter washing the same until the pH reached 5 or higher. It should be noted that PSA used for all the following experiments was prepared by the above method.

More specifically, the enzymatic reaction was carried out by first preincubating a mixture solution composed of 50 µL of 200 mM phosphoric acid buffer (pH6.0), 5 µL of the purified enzyme solution (0.1 mg/mL) and 45 µL of purified water as a reaction solution at a temperature of 90°C for 5 minutes, then additionally adding 100 µL of each substrate solution (1% by mass aqueous solutions of PSA, Avicel powder, CMC, xylan, lichenan and laminarin, and 20 mM aqueous solutions of PNPX and PNPG) that was kept at a temperature of 90°C thereto, and incubating the resulting mixture solution at a temperature of 90°C for 10 minutes. In the reaction, the mixture solution was stirred by applying vibration of 1,400 rpm using the Thermomixer (manufactured by Eppendorf) so as to avoid the precipitation of insoluble substrates.

After the completion of the reaction, in the reaction where PSA, Avicel powder, CMC, xylan, lichenan or laminarin was used as the substrate, the absorbance at 540 nm of the supernatant of the mixture solution after the reaction was measured, the amount of reduced sugar yielded by the hydrolysis was obtained, and the specific activity (U/mg) was calculated in the same manner as that of the above-mentioned process <8> where the xylanase activity of AR19G-10-6 was investigated. However, the amount of reduced sugar yielded by hydrolysis of the substrate other than xylan was obtained by using a calibration curve prepared with glucose. In the reaction where PNPG or PNPX was used as a substrate, after the completion of the reaction, the reaction was stopped by adding the same volume of a 0.2 M Na₂CO₃ solution to each mixture solution, followed by centrifugation. By so doing, the supernatant was obtained. The absorbance at 420 nm was measured by using a spectrophotometer, and the amount of p-nitrophenol in the supernatant was calculated by using a calibration curve prepared with p-nitrophenol. The amount of p-nitrophenol yielded by the enzymatic hydrolysis was obtained by the difference from the control lot. The enzymatic activity for producing 1 µmol of p-nitrophenol per minute was defined as 1U, and the value obtained by dividing it by the amount of protein mass was defined as the specific activity (U/mg).

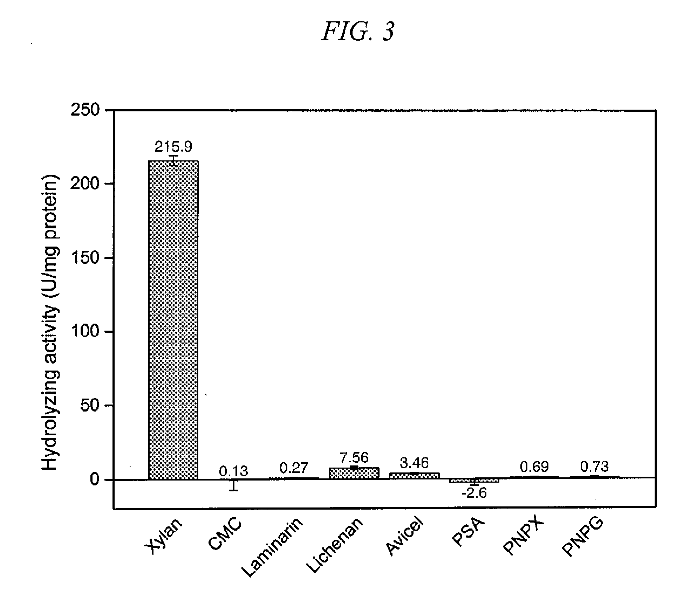

Each measurement was performed by three independent experiments, from which the mean value and the standard errors were obtained. The measurement results are shown in FIG. 3. As a result, AR19G-10-6 exhibited a high hydrolysis activity for xylan (215.9 U/mg protein). On the other hand, it exhibited almost no degradation activity for other substrates.

### <10> Temperature and pH dependencies of xylanase activity using xylan as substrate

The temperature dependency and the pH dependency of the xylanase activity of the enzyme protein (AR19G-10-6) encoded by the AR19G-10-6 gene were investigated. In the measurement, a purified enzyme solution prepared by diluting the purified enzyme (concentration of about 1 mg/mL) obtained from the above-mentioned process <7> to 0.1 mg/mL with 50 mM Tris-HCl buffer (pH8.0) was used.

The measurement of the temperature dependency of the xylanase activity of the purified AR19G-10-6 was conducted in the same manner as that of the above-mentioned process <8>, except that the reaction was carried out at a reaction temperature of 40, 50, 60, 70, 75, 80, 85, 90, 95, or 99°C, wherein the amount of reduced sugar produced by the hydrolysis was obtained, and the xylanase activity (U/mg) was calculated.

The measurement of the pH dependency of the xylanase activity of the purified AR19G-10-6 was conducted in the same manner as that of the above-mentioned process <8>, except for reacting a mixture solution composed of 100 µL of the 1% by mass xylan aqueous solution, 50 µL of McIlvaine buffer (pH3 to 8), 45 µL of purified water and 5 µL of the purified enzyme solution (0.1 mg/mL), at a temperature of 90°C for 10 minutes, wherein the amount of reduced sugar produced by the hydrolysis was obtained, and the xylanase activity (U/mg) was calculated.

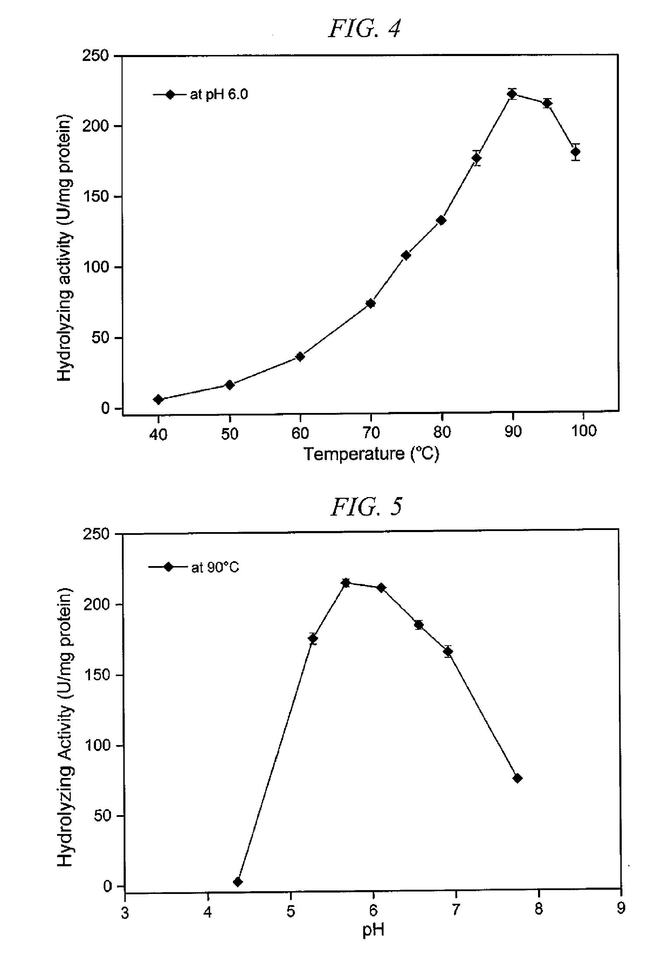

The measurement results are shown in FIGs. 4 and 5. FIG. 4 is a diagram showing the measurement results of the xylanase activity (pH6.0) of the purified enzyme AR19G-10-6 at respective temperatures, wherein the horizontal axis represents the temperature. FIG. 5 is a diagram showing the measurement results of the xylanase activity (90°C) of the purified enzyme AR19G-10-6 at respective pH values, wherein the horizontal axis represents the pH. The pH was plotted by the actual measurement values of the mixture solution containing the substrate, the buffer, and the enzyme.

The purified enzyme of AR19G-10-6 exhibited xylanase activity in a temperature range from 40 to 99°C (FIG. 4). The optimum temperature (Tₒₚₜ) showing the highest activity was 90°C at a pH of 6.0. A large reduction in the xylanase activity of AR19G-10-6 was not observed even when the enzymatic reaction temperature was set to 95°C or higher, and not less than 80% of the activity in the optimum temperature was maintained even at a temperature of 99°C.

In addition, the purified enzyme of AR19G-10-6 exhibited xylanase activity within a range of pH4.0 to 8.0 at the reaction temperature of 90°C. The optimum pH of the purified enzyme AR19G-10-6 was a pH of 5.7 at 90°C (the actual measurement value of the mixture solution containing the substrate, the buffer, and the enzyme).

<11> Thermal stability measurement of xylanase by differential scanning fluorimetry

Differential scanning fluorimetry (DSF) is one of the methods of measuring the thermal denaturation of proteins using a fluorescent dye and real-time PCR machine, and can be applied to various proteins. The fluorescent dyes used in the DSF such as SYPRO Orange emit fluorescence in nonpolar conditions when binding to the hydrophobic site, while the emission is suppressed in the polar conditions when dissolved in water. Usually, the protein structure is unfolded in the thermal denaturation temperature, and the hydrophobic regions of the protein are exposed to the protein surface. When SYPRO Orange binds to this exposed hydrophobic region, by the excitation light having a wavelength of 470 to 480 nm, strong fluorescence having a peak near a wavelength of 595 nm is emitted. By increasing the temperature of the protein solution at regular intervals in a stepwise manner and measuring the fluorescence intensity, the thermal degradation temperature (= change point of the fluorescence intensity) is calculated.

In the measurement, the purified enzyme (1 mg/mL) obtained from the above-mentioned process <7> was used.

More specifically, 2 µL of 100-fold diluted SYPRO Orange (manufactured by Life Technologies Inc.), 1 µL of the purified enzyme with a concentration of 1 mg/mL, 5 µL of 200 mM phosphoric acid buffer (pH 6.0) and 12 µL of purified water were added into the wells of a 96-well PCR plate (Multiplate 96 Well PCR Plate MLL-9651, manufactured by Bio-Rad Laboratories, Inc.) so that the volume of each well was 20 µL. The PCR plate was sealed with Optical Flat 8-Cap Strips (manufactured by Bio-Rad Laboratories, Inc.), the temperature of the well was increased by 0.2°C from 30°C up to 100°C by a real-time PCR machine (CFX96 Touch Real-Time PCR System, manufactured by Bio-Rad Laboratories, Inc.), and following a lapse of 10 seconds after the target temperature was achieved, the fluorescence intensity of each well was measured simultaneously. SYPRO Orange was excited by a light emitting diode (LED) having a wavelength range of 450 to 490 nm, the SYPRO Orange emitted light was passed through a band pass filter of 560 to 580 nm range, the measurement of the fluorescence intensity was performed with a CCD camera, and changes in the fluorescence intensity were plotted as a function of temperature. The data analysis was carried out using the analysis software CFX Manager (manufactured by Bio-Rad Laboratories, Inc.) supplied with the real-time PCR machine. Each measurement was performed by three independent experiments.

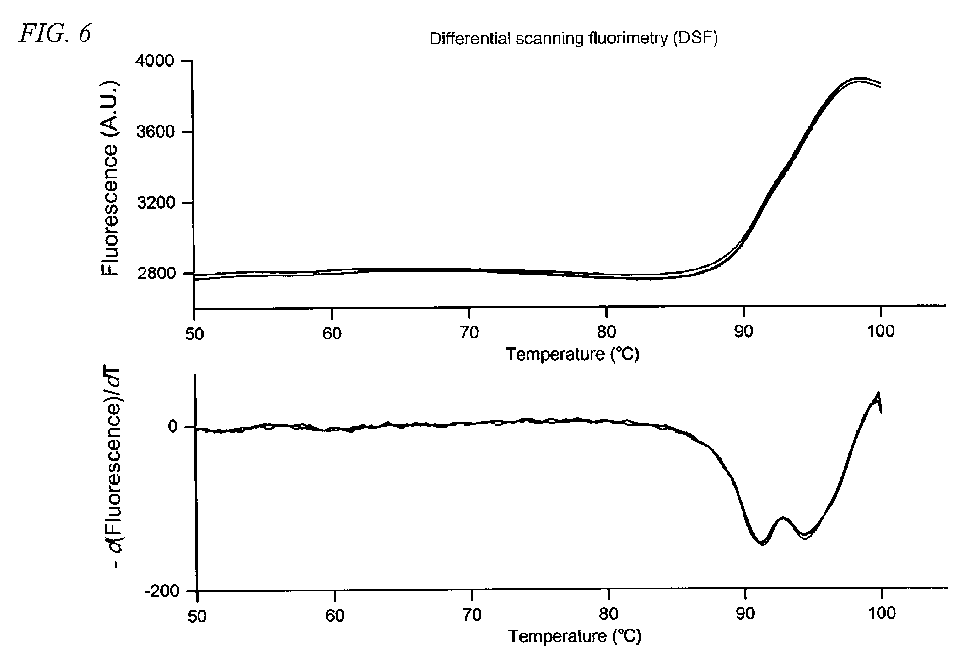

FIG. 6 shows changes in the fluorescence intensity of SYPRO Orange measured by the DSF method and caused in association with the thermal denaturation that were exhibited by the AR19G-10-6 enzymatic protein. The upper graph in FIG. 6 shows measured data, and the lower graph in FIG. 6 shows the first derivative "-*d*(Fluorescence)/*dt*" of the fluorescence intensity change curve in the upper graph of FIG. 6. The thermal denaturation temperature (melting temperature; Tm value) was defined as the local maximum value of the first derivative ("-*d*(Fluorescence)/*dt*" shown on the Y axis of the lower graph in FIG. 6) of the fluorescence intensity curve that is a function of temperature. The first derivative of the fluorescence intensity of the AR19G-10-6 enzymatic protein showed peaks at around 91°C and 94°C, suggesting that the thermal denaturation occurred at these temperatures. The average Tm values of the thermal denaturation temperature of the AR19G-10-6 enzymatic protein were 91.1°C ± 0.1 and 94.3°C ± 0.1 (n = 3), respectively, which were close to the optimum temperature Tₒₚₜ = 90°C of this enzyme obtained from the xylanase activity.

## Claims

1. A thermostable xylanase comprising a xylanase catalytic domain, the xylanase catalytic domain comprising:
(A) a polypeptide comprising an amino acid sequence represented by SEQ ID NO: 1;
(B) a polypeptide comprising an amino acid sequence in which at least one amino acid is deleted, substituted, or added in the amino acid sequence represented by SEQ ID NO: 1, and having xylanase activity at least under conditions of a temperature of 90°C and a pH of 6.0; or
(C) a polypeptide comprising an amino acid sequence having at least 75% sequence identity with the amino acid sequence represented by SEQ ID NO: 1, and having xylanase activity at least under conditions of a temperature of 90°C and a pH of 6.0.

2. The thermostable xylanase according to Claim 1, which exhibits xylanase activity in a pH of 6.0 and a temperature of 60 to 99° C.

3. A polynucleotide comprising a region that encodes a xylanase catalytic domain which comprises:
(a) a nucleotide sequence that encodes a polypeptide comprising an amino acid sequence represented by SEQ ID NO: 1;
(b) a nucleotide sequence that encodes a polypeptide comprising an amino acid sequence in which at least one amino acid is deleted, substituted, or added in the amino acid sequence represented by SEQ ID NO: 1, and having xylanase activity at least under conditions of a temperature of 90°C and a pH of 6.0;
(c) a nucleotide sequence that encodes a polypeptide comprising an amino acid sequence having at least 75% sequence identity with the amino acid sequence represented by SEQ ID NO: 1, and having xylanase activity at least under conditions of a temperature of 90°C and a pH of 6.0;
(d) a nucleotide sequence having at least 75% sequence identity with a nucleotide sequence represented by SEQ ID NO: 2, and encoding a polypeptide having xylanase activity at least under conditions of a temperature of 90°C and a pH of 6.0; or
(e) a nucleotide sequence of a polynucleotide which hybridizes with a polynucleotide comprising the nucleotide sequence represented by SEQ ID NO: 2 under a stringent condition, and being a nucleotide sequence that encodes a polypeptide having xylanase activity at least under conditions of a temperature of 90°C and a pH of 6.0.

4. The polynucleotide according to Claim 3, wherein said polypeptide exhibits xylanase activity in a pH of 6.0 and a temperature of 60 to 99° C.

5. An expression vector, which is incorporated with the polynucleotide according to Claim 3 or Claim 4, and
which is able to express a polypeptide having xylanase activity in a host cell.

6. A transformant, which is introduced with the expression vector according to Claim 5.

7. The transformant according to Claim 6, which is a eukaryotic microbe.

8. A method for producing a thermostable xylanase, comprising producing a thermostable xylanase in the transformant according to Claim 6 or 7.

9. A glycoside hydrolase mixture, comprising the thermostable xylanase according to Claim 1 or 2, a thermostable xylanase encoded by the polynucleotide according to Claim 3 or 4, or a thermostable xylanase produced by the method for producing a thermostable xylanase according to Claim 8, and at least one or more types of other glycoside hydrolases.

10. A method for producing a lignocellulose degradation product, comprising producing a lignocellulose degradation product by bringing a material composed of lignocellulose containing hemicellulose including xylan into contact with the thermostable xylanase according to Claim 1 or 2, a thermostable xylanase encoded by the polynucleotide according to Claim 3 or 4, the transformant according to Claim 6 or 7, a thermostable xylanase produced by the method for producing a thermostable xylanase according to Claim 8, or the glycoside hydrolase mixture according to Claim 9.
